# EUROPÄISCHE PATENTSCHRIFT

(11) **EP 2 926 683 B1**
(45) Veröffentlichungstag und Bekanntmachung des Hinweises auf die Patenterteilung: **26.12.2018**
(21) Anmeldenummer: 15160230.7
(22) Anmeldetag: 23.03.2015
(51) Int. Cl.: A45D 24/26, A45D 34/04

(54) **Innengespeister Applikator mit Fingern und Folienvorratsbehälter**
Internally fed applicator with fingers and film storage container
Applicateur alimenté par l'intérieur, doté de doigts et récipient de stockage de feuilles

(30) Priorität: 03.04.2014 DE 202014002986 U
(43) Veröffentlichungstag der Anmeldung: 07.10.2015
(73) Patentinhaber: GEKA GmbH, 91572 Bechhofen (DE)
(72) Erfinder: Hauger, Christian, 78166 Donaueschingen (DE)
(74) Vertreter: Misselhorn, Hein-Martin

(56) Entgegenhaltungen:
- WO-A1-2013/156648
- DE-A1- 3 931 111
- DE-U1-202010 007 365
- US-A1- 2011 174 835
- US-B1- 6 302 607

## Beschreibung

Innengespeiste Applikatoren sind aus zum Beispiel US6302707B1 bekannt.

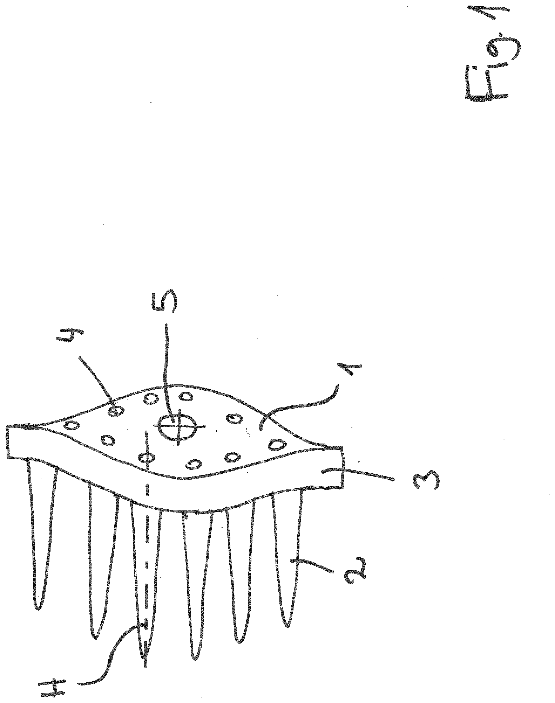

Die Figur 1 zeigt einen Fingerträger 1, der zu einem ersten Ausführungsbeispiel gehört. Dieser Fingerträger ist hier als eine Platte (fingertragende Platte) ausgestaltet, die vorzugsweise zumindest im Wesentlichen und idealerweise vollständig senkrecht zur späteren Längsachse L des Applikators verläuft.
Diese fingertragende Platte weist einen Außenumfang 3 auf, der vorzugsweise glattflächig und etwa mindestens 0,5 cm, besser mindestens 0,75 cm breit in Richtung parallel zur Längsachse L ist. Dieser Außenumfang 3 dient zum Anschweißen des in Figur 1 nicht gezeigten Folienbehälters.
Der Fingerträger kann einen zentralen Speisekanal 5 aufweisen, der dazu dient, um zu applizierende Substanz von der Seite, die in Figur 1 dem Betrachter zugewandt ist, auf die andere Seite, die mit den Borsten 2 besetzt ist, zu leiten, um sie dort zur Applikation zur Verfügung zu stellen. Zusätzlich oder stattdessen kann mindestens eine Borste innen hohl sein und daher einen Borstenkanal 4 aufweisen, der die gleiche Funktion besitzt.
Besonders bevorzugt ist es, wenn der Fingerträger in Richtung senkrecht zur Längsachse L eine schiffchenförmige Gestalt aufweist, wie es die Figur 1 veranschaulicht. Unter einer schiffchenförmige Gestalt wird eine Gestalt verstanden, die zumindest an 2 Stellen spitz zuläuft und vorzugsweise im Bereich der Spitzen nicht mehr als 2 mm, bzw. besser nicht wesentlich mehr als eine Lage der den Folienbehälter bildenden Folie 21 dick ist. Unter "wesentlich mehr" wird hier maximal 30 %, besser maximal 20 % verstanden. Auf diese Art und Weise wird es besonders einfach, die Folie mit dem Fingerträger zu verschweißen.

Schon der von Figur 1 gezeigte Fingerträger kann zusätzlich einen integralen Rohrstutzen aufweisen, der hier in Figur 1 aber nicht zeichnerisch dargestellt ist und der später im Zusammenhang mit anderen Ausführungsbeispielen noch näher gezeigt wird. In diesem Fall kann die den Folienbehälter bildende Folie auch mit dem Rohrstutzen verschweißt sein, anstatt unmittelbar mit dem Außenumfang der fingertragenden Platte.
Um einen möglichst preisgünstigen Applikator auszubilden, wird der von Figur 1 gezeigte Fingerträger in einen Folienbehälter eingeführt, der vorzugsweise als in Umfangsrichtung nahtlos extrudierter oder geblasener Folienschlauch 6 ausgebildet ist und der erfindungsgemäß eine Wandstärke von maximal 0,25 mm aufweist. Für diese Dickenangabe sind allerdings lokale Dickstellen, wie sie zum Beispiel durch eine Verschweißung zweier Folien miteinander gebildet werden, irrelevant.

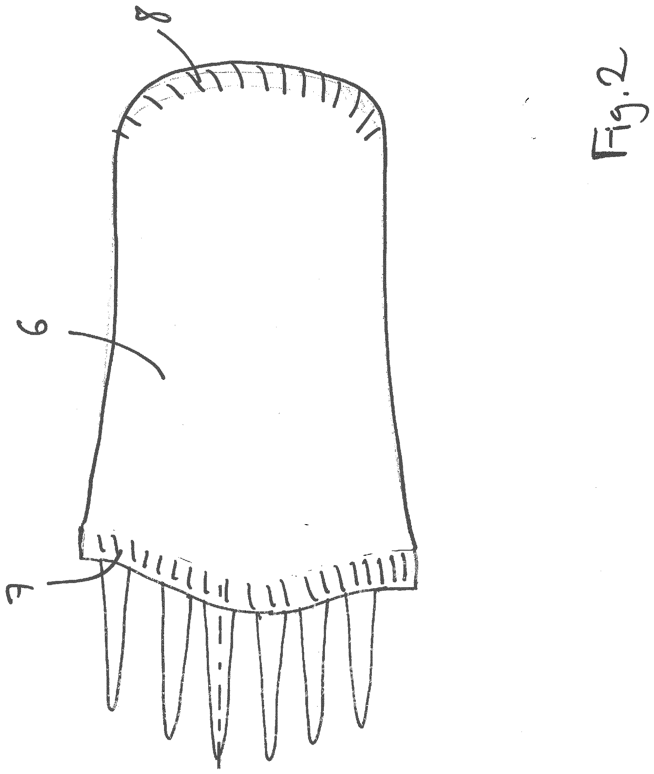

Wie das Ganze im fertigen Zustand aussieht, ist sehr gut in Figur 2 gezeigt. Hier sieht man die Schweißnaht 7, mit der der Folienschlauch unmittelbar an den Außenumfang 3 des in Figur 1 dargestellten Fingerträgers angeschweißt ist. Auf der dem Fingerträger abgewandten Seite ist der Folienschlauch zunächst offen, von hier her wird er befüllt. Nach dem Befüllen wird der Folienschlauch auf der der fingertragenden Platte abgewandten Seite zusammengedrückt und verschweißt, dies ergibt die in Figur 2 zu sehende Schweißnaht 8.
Generell ist zu den hier in Rede stehenden Fingerträgern festzuhalten, dass diese im Wesentlichen in sich biegesteif sind. Anders als die Folie, die den Folienbehälter bildet, ist der Fingerträger daher nicht knautschbar, sondern erfährt - abgesehen von seinen Borsten - unter den bei der Applikation auftretenden Kräften allenfalls Verformungen, die im Bereich unterhalb von 5/10 mm liegen. Diese Definition gilt für alle der hier beschriebenen Fingerträger. Zu dem Folienbehälter ist noch festzuhalten, dass er sich aufgrund der geringen Dicke der Folie meist dadurch auszeichnet, dass er knautschbar ist, d.h. der Benutzer oder die Benutzerin kann den Folienbehälter so zusammendrücken, dass sich der Folienbehälter mit einer unbestimmten Zahl von Falten zusammenlegt.
Zu dem Fingerträger ist noch festzuhalten, dass unter Fingern im Sinne der Erfindung vorzugsweise Borsten verstanden werden. Borsten sind stabartige Auftragsorgane, deren Länge mindestens 5mal größer als ihr maximaler Durchmesser oberhalb derjenigen Verrundung ist, mit der diese in den Borstenträger übergehen mögen. Erfindungsgemäß sind die Borsten derart flexibel, dass sie sich um einen Betrag von mindestens dem Sechsfachen des maximalen Borstendurchmessers senkrecht zur Borstenlängsachse H im unbelasteten Zustand seitlich auslenken lassen, ohne eine plastische Verformung zu erleiden. Dieser Hinweis zum Fingerträger gilt für alle Ausführungsbeispiele. Die bevorzugt verwendeten, spritzgegossenen Borsten haben ein bestimmtes körperliches Merkmal, welches sie von anderen Borsten unterscheidet, so dass sich hier die Herstellung in den körperlichen Merkmalen, die bei einer Borste anzutreffen sind, niederschlägt. Beim Spritzgießen von Borsten richten sich nämlich in den engen borstenformenden Kanälen die Molekülketten des die borstenbildenden Kunststoffs weitgehend parallel aus, was einer Borste eine entsprechende Struktur und wesentlich verbesserte Applikationseigenschaften verleiht, insbesondere was das Wiederaufrechtvermögen angeht. Alternativ können auch anderweitig befestigte Borsten zum Einsatz kommen, z. B. in bekannter Weise getuftete Borsten.

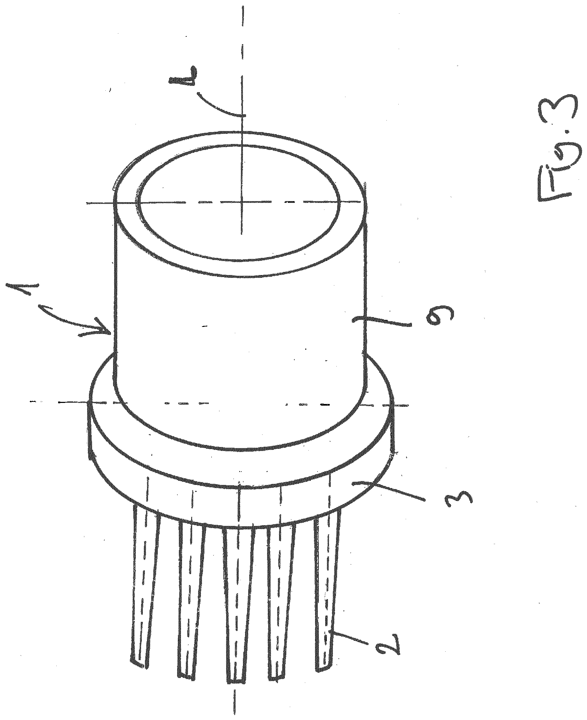

Die Figur 3 zeigt ein anderes Ausführungsbeispiel für den Fingerträger. Dieser Fingerträger zeichnet sich durch seine runde Gestalt aus und dadurch, dass er integral in einen Rohrstutzen 9 übergeht. Gut zu erkennen ist die Längsachse L des Fingerträgers. Nicht zu erkennen ist der Speisekanal 5, ebenfalls nicht zu erkennen sind der oder die Borstenkanäle 4, die stattdessen oder zusätzlich mindestens durch eine Borste hindurch gehen und es erlauben, die zu applizierende Substanz über die Borsten von einer Seite des Fingerträgers auf dessen andere Seite auszugeben. Diese Einrichtungen sind aber auch bei diesem Ausführungsbeispiel vorhanden.

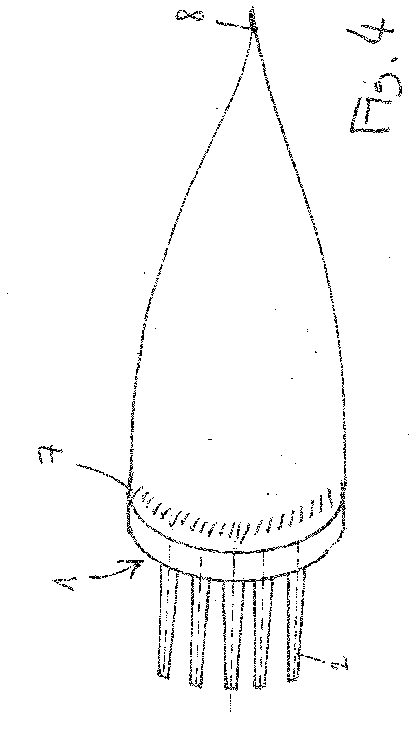

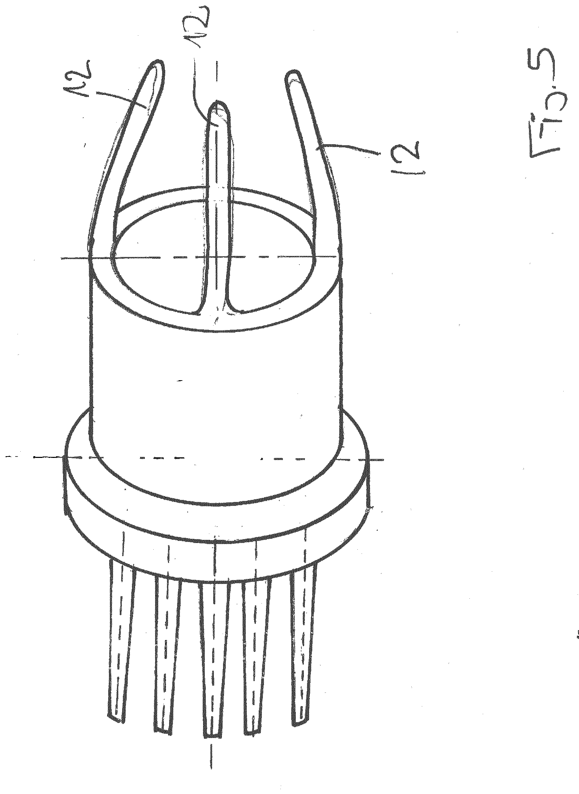

Der Rohrstutzen macht es entscheidend einfacher, den Fingerträger in einen Folienschlauch einzuführen und mit diesem zuverlässig dicht zu verbinden. Zu diesem Zweck wird der Folienschlauch kurzerhand ähnlich wie ein Strumpf auf den Rohrstutzen aufgezogen bzw. aufgeschoben und dann entweder nicht erfindungsgemäß mit dem Rohrstutzen verschweißt, wie das bei Figur 4 gezeigt ist, oder erfindungsgemäß unmittelbar mit der fingertragenden Platte des Fingerträgers, so wie das bereits im Rahmen des Ausführungsbeispiels 1 geschildert worden ist.
Die Figur 4 zeigt den auf diese Art und Weise entstehenden fertigen Applikator.
Eine Variante zur Verwirklichung dieses Ausführungsbeispiels zeigt die Figur 5, hier geht der Rohrstutzen in mehrere elastische, vorzugsweise sich schräg einwärts zur Längsachse L erstreckende Spreizarme 12 über. Diese Spreizarme 12 erleichtern das Einführen des Fingerträgers in den Folienschlauch, denn sie dienen hier als Führungskufen, die den Folienschlauch aufspreizen. Gleichzeitig können diese Spreizarme 12 auch den Zweck haben, dass sie den fertig montierten und befüllten Folienschlauch in einer ansehnlichen Art und Weise aufgespreizt halten. Welche Funktion diese Spreizarme übernehmen, hängt von ihrer Länge ab. Die Spreizarme sind vorzugsweise integraler Bestandteil des Rohrstutzens, wo ein Rohrstutzen fehlt, können sie integraler Bestandteil des Fingerträgers bzw. der fingertragenden Platte sein.

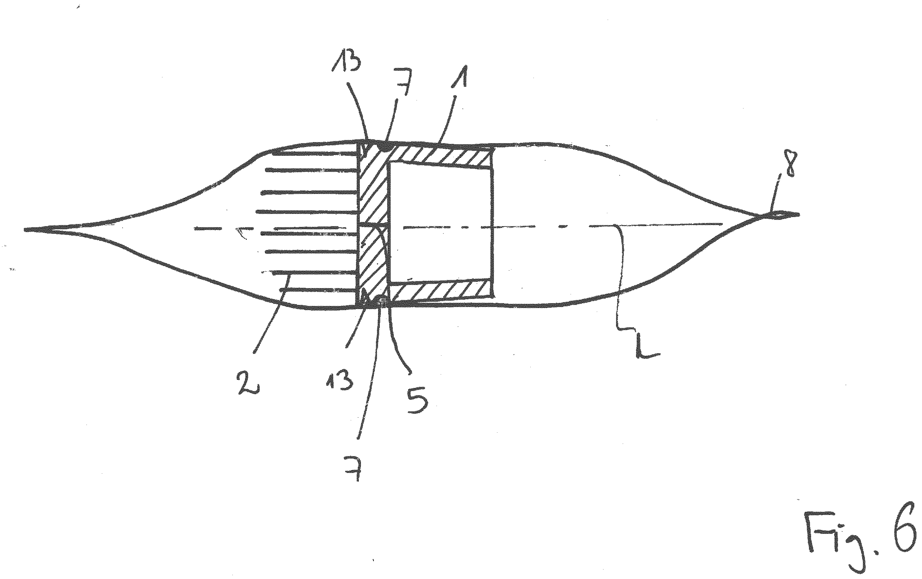

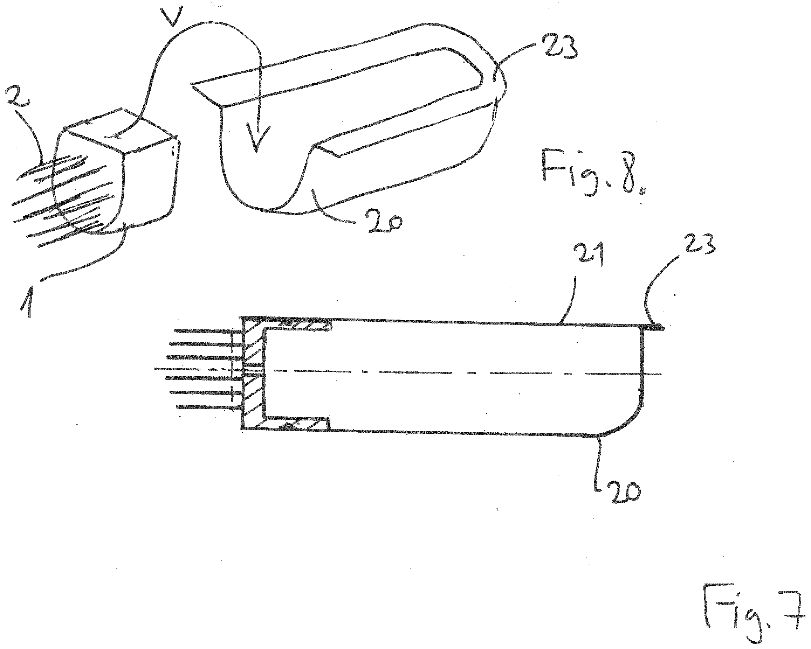

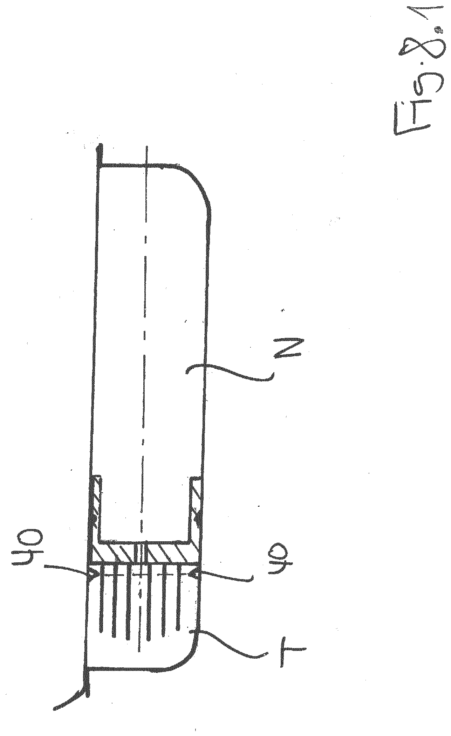

Die Figur 6 zeigt ein weiteres Ausführungsbeispiel, das von dem soeben beschriebenen Ausführungsbeispiel abgeleitet ist und mit diesem relativ eng verwandt ist. Auch hier ist ein vorzugsweise kreisrunder Fingerträger vorgesehen, der integral in einen Rohrstutzen übergeht. Wie man hier relativ gut sieht, kann der Rohrstutzen in Richtung seines dem Fingerbesatz abgewandten Endes leicht kegelig eingezogen sein, um auf diese Art und Weise sein Einführen in den Folienschlauch zu erleichtern. Im Gegensatz zu dem von Figur 4 gezeigten Ausführungsbeispiel wird der Fingerträger im Zuge der Herstellung so tief in den Folienschlauch eingeschoben, dass dieser auch die Finger des Fingerträgers überragt. Der Folienschlauch kann dann an beiden Enden zugeschweißt werden und bildet auf diese Art und Weise nicht nur den Vorratsbehälter zur Bevorratung der aufzutragenden Substanz, sondern zugleich auch noch einen Schutz für die Finger, der den hygienischen Zustand der Finger bis zum Anbruch garantiert. Zu diesem Zweck kann der Folienschlauch an entsprechenden Stellen, entweder im Bereich des Fingerträgers, wie hier gezeigt, oder aber auch im Anschluss an den Fingerträger mit Dünnstellen, Kerben oder ähnlichen Hilfsmitteln versehen werden, die eine Sollbruchstelle 13 darstellen, an der die Folie abreißt, um den Fingerbesatz freizugeben, wenn man an ihrem auf der Seite des Fingerbesatzes liegenden Ende zieht. Die Figur 7 zeigt ein gänzlich anderes Ausführungsbeispiel der Erfindung. Zum Einsatz kommt hier abermals der erfindungsgemäße Fingerträger 1. Vorzugsweise hat auch hier der Fingerträger 1 einen integral angeformten Rohrstutzen 9. Der Vorratsbehälter für die zu applizierende Substanz ist bei diesem Ausführungsbeispiel gänzlich anders gestaltet. Der Vorratsbehälter wird hier durch einen Blister gebildet, der aus einem Tray 20 besteht und einer den Tray versiegelnden Folie 21. Der Tray weist die Besonderheit auf, dass er an einer Stirnseite offen ist, so, wie das die Figur 8 zeigt. In diese offene Stirnseite wird dann der Fingerträger 1 durch eine Bewegung in Richtung des Pfeils V eingesetzt. Da die Außenkontur, d.h. die Kontur des Außenumfangs des Fingerträgers, dem Querschnittsprofil des Tray 20 entspricht, kann der Fingerträger leicht mit dem Tray 20 verklebt oder verschweißt werden. Auf diese Art und Weise bildet dann der Tray eine mit Ausnahme an seiner Oberseite komplett geschlossene Mulde, die dazu prädestiniert ist, das zu applizierende Kosmetikum aufzunehmen. Da der Fingerträger idealerweise auf seiner Oberseite eine Fläche ausbildet, die mit den Flanschflächen 23 des Trays nach Montage des Fingerträgers in einer Ebene liegt, kann leicht eine Folie aufgesiegelt werden, indem diese mit der Flanschflächen 23 des Tray und mit der entsprechenden Fläche des Fingerträgers verschweißt oder verklebt wird.

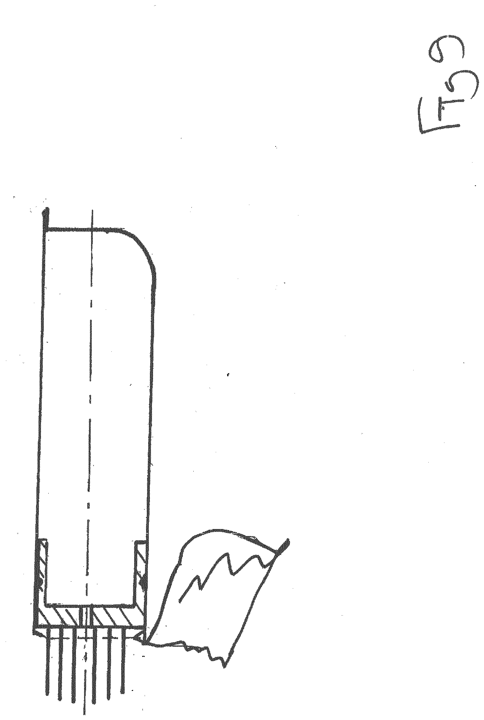

Die Figur 8 zeigt eine Abwandlung des zuletzt beschriebenen Ausführungsbeispiels. Der Unterschied zu diesem Ausführungsbeispiel, zu dem ansonsten das zu dem vorigen Ausführungsbeispiel Gesagte entsprechend gilt, ist der, dass der Tray hier so ausgeführt ist, dass er von dem Fingerträger 1 in einen Nassbereich N und einen Trockenbereich T unterteilt wird. Der Nassbereich nimmt, genau wie im vorherigen Ausführungsbeispiel, das zu applizierende Kosmetikum auf. Der Trockenbereich bildet einen Schutz für den Fingerbesatz aus, zum Beispiel, um diesen bis zum Anbruch hygienisch einwandfrei bevorratet zu halten. Der Tray 20 ist als ganzer, auch im Bereich des Fingerbesatzes mit der Folie 21 versiegelt, die auch hier wieder gegen die Flanschflächen 23 des Tray verschweißt ist, der nun allerdings komplett umläuft. Vorzugsweise hat der Tray eine Sollbruchstelle, beispielsweise in Gestalt einer oder mehrerer der von Figur 8 gezeigten Kerben 40. Diese Ausgestaltung gestattet es, den Abschnitt des Tray, der den Fingerbesatz schützt, vom Rest des Tray, welcher das aufzutragende Kosmetikum enthält, abzubrechen und so weit beiseite zu klappen, dass appliziert werden kann, so, wie das die Figur 9 zeigt. Idealerweise lässt man es nicht bei einem derartigen Umklappen, sondern gestaltet den zu entfernenden Abschnitt des Tray so, dass er vollständig beseitigt werden kann.

Es gibt ein Verfahren zur Herstellung der soeben beschriebenen Applikatoren bzw. Applikatoreinheiten.

Nicht erfindungsgemäß gibt es einen innengespeisten Applikator zum Auftragen einer fließfähigen Substanz, insbesondere eines Kosmetikums oder Pharmazeutikums, mit einem festen Fingerträger 1 mit angespritzten Fingern und mindestens einem Speisekanal zur Leitung der Substanz von der den Fingern abgewandten Seite des Fingerträgers 1 auf die mit den Fingern besetzte Seite des Fingerträgers 1, wobei sich an die den Fingern abgewandte Seite des Fingerträgers 1 (knautschbarer) ein Blister aus einem Tray 20 und einer diesen versiegelnden Folie 21 anschließt, der einen Vorrat der aufzutragenden Substanz enthält, wobei der Anschluss derart ist, dass durch Druck auf den Tray 20 und/oder die den Tray 20 versiegelnde Folie 21 ein Volumen der aufzutragenden Substanz durch den Speisekanal hindurch ausgegeben wird.

Es gibt ebenfalls einen innengespeisten Applikator, der sich dadurch auszeichnet, dass der Tray 20 und vorzugsweise auch die den Tray 20 versiegelnde Folie 21 mit dem Fingerträger 1 verschweißt ist. Weiterhin gibt es einen innengespeisten Applikator, der sich dadurch auszeichnet, dass der Fingerträger 1 den Tray 20 in einen Trocken- und einen Nassbereich N unterteilt und der Trockenbereich T den Fingerbesatz des Fingerträgers 1 aufnimmt, wobei der Tray 20 bevorzugt so gestaltet ist, dass er im Trockenbereich T eine Sollbruchstelle 13 aufweist, die es erlaubt, vor Benutzung den Teil des Trays 20, der bis zur Benutzung den Fingerbesatz schützt, abzubrechen und vollständig oder so zu entfernen, dass mit Hilfe des Fingerbesatzes aufgetragen werden kann.
Im o.g. Sinne gibt es einen innengespeisten Applikator, der sich dadurch auszeichnet, dass der Fingerträger 1 den Tray 20 in einen Trocken- und einen Nassbereich N unterteilt und der Trockenbereich T den Fingerbesatz des Fingerträgers 1 aufnimmt, wobei der Tray 20 bevorzugt so gestaltet ist, dass er im Trockenbereich T hinreichend dünnwandig ist, um dort derart zur Seite umgestülpt werden zu können, dass mit Hilfe des Fingerbesatzes aufgetragen werden kann.

### Bezugszeichenliste

- 1: Fingerträger
- 2: Borsten
- 3: Außenumfang
- 4: Borstenkanal
- 5: zentraler Speisekanal
- 6: Folienschlauch
- 7: Schweißnaht am Fingerträger
- 8: Schweißnaht an Fingerträger abgewandten Seite
- 9: Rohrstutzen
- 10: nicht vergeben
- 11: nicht vergeben
- 12: Spreizarme
- 13: Sollbruchstelle
- 14: nicht vergeben
- ⋮:
- 19: nicht vergeben
- 20: Tray
- 21: Folie
- 22: nicht vergeben
- 23: Flanschflächen
- 24: nicht vergeben
- ⋮:
- 39: nicht vergeben
- 40: Kerben
- L: Längsachse
- N: Nassbereich
- T: Trockenbereich
- H: Borstenlängsachse
- V: Bewegung in Richtung des Pfeils V

## Patentansprüche

1. Innengespeister Applikator zum Auftragen eines Kosmetikums oder Pharmazeutikums, wobei der Applikator einen festen Fingerträger (1) mit angespritzten Fingern und mindestens einem Speisekanal zur Leitung der Substanz von der den Fingern abgewandten Seite des Fingerträgers (1) auf die mit den Fingern besetzte Seite des Fingerträgers (1) besitzt, wobei sich an die den Fingern abgewandte Seite des Fingerträgers ein Folienbehälter aus einer Folie mit einer Wandstärke von maximal 0,25 mm der einen Vorrat der aufzutragenden Substanz enthält, derart anschließt, dass durch Druck auf den Folienbehälter, dessen Folie im fertigen Zustand die Behälteraußenwand bildet, ein Volumen der aufzutragenden Substanz durch den Speisekanal hindurch ausgegeben wird, wobei der Folienbehälter mit dem Außenumfang (3) der durch den Fingerträger aufgewiesenen fingertragenden Platte verschweißt ist und wobei die Finger Borsten sind, die sich um den Betrag von mindestens dem Sechsfachen des maximalen Borstendurchmessers senkrecht zur Position der Borstenlängsachse in unbelastetem Zustand seitlich auslenken lassen, ohne dass sie eine plastische Verformung erleiden wobei der Fingerträger (1) in mindestens zwei, besser mindestens vier Spreizarme übergeht zum Aufgespreitzthalten des Folienbehälters.

2. Innengespeister Applikator nach Anspruch 1, **dadurch gekennzeichnet, dass** der Fingerträger (1) in sich im Wesentlichen biegesteif ist.

3. Innengespeister Applikator nach einem der vorhergehenden Ansprüche, **dadurch gekennzeichnet, dass** der Folienbehälter nur mit einer Umfangsfläche des Fingerträgers (1) verschweißt ist.

4. Innengespeister Applikator nach einem der vorhergehenden Ansprüche, **dadurch gekennzeichnet, dass** der Folienbehälter ein über seinen Außenumfang (3) hinweg nahtlos geblasener oder extrudierter Folienschlauch (6) ist.

5. Innengespeister Applikator nach einem der vorhergehenden Ansprüche, **dadurch gekennzeichnet, dass** der Folienschlauch (6) an seinem dem Fingerträger (1) abgewandten Ende in sich verschweißt ist.

6. Innengespeister Applikator nach einem der vorhergehenden Ansprüche, **dadurch gekennzeichnet, dass** der Fingerträger (1) aus der fingertragenden Platte besteht, die auf ihrer den Fingern abgewandten Seite integral in einen Rohrstutzen (9) übergeht, den der Folienbehälter und insbesondere der Folienschlauch (6) strumpfartig übergreift.

7. Innengespeister Applikator nach einem der vorhergehenden Ansprüche, **dadurch gekennzeichnet, dass** der Rohrstutzen (9) sich zu seiner dem Fingerträger (1) abgewandten Seite hin verjüngt, vorzugsweise indem er eine kegelige Gestalt aufweist.

8. Innengespeister Applikator nach einem der vorhergehenden Ansprüche, **dadurch gekennzeichnet, dass** der Fingerträger (1) oder dessen Rohrstutzen (9) in mindestens vier Spreizarme übergeht.

9. Innengespeister Applikator nach einem der vorhergehenden Ansprüche, **dadurch gekennzeichnet, dass** der Borstenträger eine schiffchenartige Gestalt hat.

10. Innengespeister Applikator nach einem der vorhergehenden Ansprüche, **dadurch gekennzeichnet, dass** der Fingerträger (1) ein Gewinde zum Aufschrauben einer Schraubkappe aufweist oder eine Rasteinrichtung zum Aufrasten einer Kappe.

11. Innengespeister Applikator nach einem der vorhergehenden Ansprüche, **dadurch gekennzeichnet, dass** der Folienschlauch (6) den Fingerträger (1) auch auf der Seite von dessen Fingern übergreift und vorzugsweise an seinem dortigen Ende in sich verschweißt ist.

12. Applikatoreinheit bestehend aus mindestens einem Applikator nach einem der vorhergehenden Ansprüche, **dadurch gekennzeichnet, dass** der Applikator in einen Blister aus einem Tray (20), mit einer den Applikator aufnehmenden Vertiefung eingelegt ist und zumindest die den Applikator aufnehmende Vertiefung, besser der ganze Tray (20) einseitig mit einer Folie (21) versiegelt ist.

## Claims

1. An internally supplied applicator for applying a cosmetic or pharmaceutical, wherein the applicator has a solid finger carrier (1) with molded-on fingers and at least one supply conduit for conducting the substance from the side of the finger carrier (1) facing away from the fingers to the side of the finger carrier (1) covered by the fingers, wherein a film container composed of a film with a wall thickness of at most 0.25 mm containing a supply of the substance to be applied adjoins the side of the finger carrier facing away from the fingers in such a way that a volume of the substance to be applied is dispensed through the supply conduit by pressing on the film container, whose film forms the outer container wall in the finished state, wherein the film container is welded to the outer circumference (3) of the finger-carrying plate that the finger carrier has, and wherein the fingers are bristles that can be laterally deflected in the unstressed state by the amount of at least six times the maximum bristle diameter perpendicular to the position of the longitudinal bristle axis without suffering plastic deformation, wherein the finger carrier (1) transitions into at least two, better at least four, spreading arms in order to keep the film container spread open.

2. The internally supplied applicator according to claim 1, **characterized in that** the finger carrier (1) is substantially inherently rigid.

3. The internally supplied applicator according to any one of the preceding claims, **characterized in that** the film container is welded only to a circumferential surface of the finger carrier (1).

4. The internally supplied applicator according to any one of the preceding claims, **characterized in that** the film container is a film tube (6) blown or extruded so that it is seamless throughout its outer circumference (3).

5. The internally supplied applicator according to any one of the preceding claims, **characterized in that** the film tube (6) is welded to itself at the end thereof facing away from the finger carrier (1).

6. The internally supplied applicator according to any one of the preceding claims, **characterized in that** the finger carrier (1) is composed of the finger-carrying plate which, on the side thereof facing away from the fingers, transitions integrally into a pipe socket (9) over which the film container, and particularly the film tube (6), extends like a stocking.

7. The internally supplied applicator according to any one of the preceding claims, **characterized in that** pipe socket (9) tapers towards the side thereof facing away from the finger carrier (1), preferably by having a conical shape.

8. The internally supplied applicator according to any one of the preceding claims, **characterized in that** the finger carrier (1) or its pipe socket (9) transitions into at least four spreading arms.

9. The internally supplied applicator according to any one of the preceding claims, **characterized in that** the bristle carrier has a boat-like shape.

10. The internally supplied applicator according to any one of the preceding claims, **characterized in that** the finger carrier (1) has a thread for screwing on a screw cap, or a snap-on device for snapping on a cap.

11. The internally supplied applicator according to any one of the preceding claims, **characterized in that** the film tube (6) extends over the finger carrier (1) also on the side of the latter's fingers, and is preferably welded to itself at its end there.

12. An applicator unit composed of at least one applicator according to any one of the preceding claims, **characterized in that** the applicator is placed into a blister composed of a tray (20), with a depression accommodating the applicator, and at least the depression accommodating the applicator, better the entire tray (20), is sealed with a film (21) on one side.

## Revendications

1. Applicateur alimenté par l'intérieur pour appliquer un produit cosmétique ou pharmaceutique, l'applicateur comportant un porte-doigts (1) fixe doté de doigts moulés par injection et d'au moins un canal d'alimentation pour amener la substance depuis le côté du porte-doigts (1) détourné des doigts jusqu'au côté du porte-doigts (1) doté des doigts,
dans lequel
au côté du porte-doigts détourné des doigts se raccorde un récipient en feuille constitué d'une feuille d'une épaisseur de paroi de 0,25 mm au maximum, qui contient une réserve de la substance à appliquer, de telle sorte que par une pression exercée sur le récipient en feuille dont la feuille constitue la paroi extérieure du récipient, à l'état fini, un volume de la substance à appliquer est distribué à travers le canal d'alimentation,
le récipient en feuille est assemblé par soudage à la périphérie extérieure (3) de la plaque formée par le porte-doigts et portant les doigts, et
les doigts sont des poils qui, dans l'état non chargé, se laissent débattre latéralement de la valeur d'au moins six fois le diamètre maximal des poils, perpendiculairement à la position de l'axe longitudinal des poils, et ceci sans subir une déformation plastique,
le porte-doigts (1) se transforme en au moins deux, mieux en au moins quatre bras d'écartement pour maintenir écarté le récipient en feuille.

2. Applicateur alimenté par l'intérieur selon la revendication 1,
**caractérisé en ce que**
le porte-doigts (1) est en lui-même sensiblement rigide en flexion.

3. Applicateur alimenté par l'intérieur selon l'une des revendications précédentes,
**caractérisé en ce que**
le récipient en feuille n'est assemblé par soudage qu'avec une surface périphérique du porte-doigts (1).

4. Applicateur alimenté par l'intérieur selon l'une des revendications précédentes,
**caractérisé en ce que**
le récipient en feuille est un tuyau en feuille (6) soufflé ou extrudé sans jointure sur sa périphérie extérieure (3).

5. Applicateur alimenté par l'intérieur selon l'une des revendications précédentes,
**caractérisé en ce que**
le tuyau en feuille (6) est soudé sur lui-même à son extrémité détournée du porte-doigts (1).

6. Applicateur alimenté par l'intérieur selon l'une des revendications précédentes,
**caractérisé en ce que**
le porte-doigts (1) est constitué par la plaque portant les doigts qui, sur son côté détourné des doigts, se transforme intégralement en un manchon tubulaire (9) que coiffent en forme de chaussette le récipient en feuille et en particulier le tuyau en feuille (6).

7. Applicateur alimenté par l'intérieur selon l'une des revendications précédentes,
**caractérisé en ce que**
le manchon tubulaire (9) va en se rétrécissant vers son côté détourné du porte-doigts (1), de préférence en présentant une configuration conique.

8. Applicateur alimenté par l'intérieur selon l'une des revendications précédentes,
**caractérisé en ce que**
le porte-doigts (1) ou son manchon tubulaire (9) se transforme en au moins quatre bras d'écartement.

9. Applicateur alimenté par l'intérieur selon l'une des revendications précédentes,
**caractérisé en ce que**
le porte-poils présente une configuration semblable à un fuseau.

10. Applicateur alimenté par l'intérieur selon l'une des revendications précédentes,
**caractérisé en ce que**
le porte-doigts (1) présente un pas de vis pour visser un capuchon à visser ou un moyen d'enclenchement pour enclencher un capuchon.

11. Applicateur alimenté par l'intérieur selon l'une des revendications précédentes,
**caractérisé en ce que**
le tuyau en feuille (6) coiffe le porte-doigts (1) également sur le côté de ses doigts et est de préférence soudé sur lui-même à son extrémité de ce côté.

12. Ensemble applicateur constitué par au moins un applicateur selon l'une des revendications précédentes,
**caractérisé en ce que**
l'applicateur est posé dans un blister constitué d'un plateau (20) pourvu d'une cavité recevant l'applicateur, et
au moins la cavité recevant l'applicateur, de préférence tout le plateau (20) est scellé(e) sur un côté d'une pellicule (21).
